Europäisches Patentamt

European Patent Office

Office européen des brevets

(19)

(11) Publication number: **0 271 410**
**A2**

# EUROPEAN PATENT APPLICATION

(12)

(21) Application number: **87402784.0**

(22) Date of filing: **08.12.87**

(51) Int. Cl.⁴: **A 61 B 3/02**

(30) Priority: **10.12.86 US 940043**

(43) Date of publication of application:
**15.06.88 Bulletin 88/24**

(84) Designated Contracting States:
**CH DE FR GB IT LI**

(71) Applicant: **Hofeldt, Albert J.**
**116 East 66th Street**
**New York New York 10021 (US)**

(72) Inventor: **Hofeldt, Albert J.**
**116 East 66th Street**
**New York New York 10021 (US)**

(74) Representative: **Bloch, Gérard et al**
**6, rue du Faubourg Saint-Honoré**
**F-75008 Paris (FR)**

(54) Illusion stereometer.

(57) A device useful in diagnosing optic nerve and ocular diseases in people who are capable of experiencing the Pulfrich stereo-illusion phenomenon, which comprises a suitably sized sphere (5) and a suitably sized cone (6), one of which is movable and the other of which is stationary, said sphere and said cone being suitably illuminated and positioned with the bottom of the sphere below the cone so that they can be seen by a person to be tested, means either for traversing the sphere slightly behind the cone or moving the cone in an arc slightly in front of the sphere at a fixed rate of movement, polarizing filters (12) capable of being adjusted with respect to the amount of light which will pass through disposed in adjustable means (15) suitable for the person to be tested to look through at a distance from said sphere and cone enabling said person to readily see said sphere and cone.

]

FIG. 1

EP 0 271 410 A2

## Description

The Pulfrich stereo-illusion is a psychophysical response found to be dependent on binocular central vision. Utilizing the Pulfrich stereo-illusion, I have developed a testing technique for determining the presence of diseases affecting the central vision which interfere with stereopsis, the brightness of the image, or the neurosensory function of the central retina and optic nerve. The device involves controlling the variables of the observation of a sphere and a cone and the use of an 80% attenuation neutral density filter or polarizing filter which allows all normal sighted people to appreciate the normal stereo-illusion. People having good visual acuity and stereopsis, but with unequal retinal luminance or neurosensory dysfunction experience an abnormal, spontaneous stereo-illusion. People who lack stereopsis or have reduced visual acuity in at least one eye cannot appreciate the normal stereo-illusion. A testing procedure that readily identifies these two abnormal responses is achieved by utilizing my device.

The phenomenon of a frontal place oscillating object appearing to travel in an elliptical orbit when viewed with unequal binocular illumination was described by Pulfrich in 1922. Looking from above, the direction of the orbit is clockwise when the image perceived by the left eye is dimmer and counterclockwise when the image to the right eye is dimmer. The apparent depth of the orbit has been found to vary directly with the velocity of the oscillating pendulum, the difference in the binocular retinal illumination, the distance from the observer to the pendulum bob and inversely with the background illumination level.

Pulfrich postulated that the stereo-effect resulted from a prolonged latent period of vision along the afferent pathway of the eye perceiving the dimmer image. More recent studies using the episcotistor, random dot stereo-movies and visual-evoked responses, have demonstrated that the latent period of vision increases as the retinal illumination decreases. The asynchronous conduction of impulses originating from corresponding retinal points produces disparity in the simultaneous perception of these stereo-pairs and hence the false sensation of depth of the viewer.

The minimum visual and stereoscopic acuities required to appreciate the normal stereo-illusion have not heretofore been established. It has been suggested that the appreciation of the Pulfrich stereo-illusion depends not only on normal vision, but also upon the skill of the individual as an observer. Consequently, the inability to induce the normal stereo-illusion by placing a filter before one eye has not been considered a sign of visual dysfunction since factors other than visual acuity and stereopsis have been thought to influence the appreciation of this curious phenomenon.

In diseases causing an asymmetric delay in nerve conduction along the two visual pathways, the Pulfrich stereo-illusion can be appreciated without placing a filter to dim the image on one eye. This abnormal Pulfrich stereo-illusion has been studied only in retrobulbar neuritis, particularly the variety associated with multiple sclerosis. In this condition, the stereo-illusion has compared favorably with other sensory tests in detecting optic nerve involvement. In some cases, the Pulfrich stereo-illusion was more sensitive in detecting optic nerve dysfunction than color vision or the relative afferent pupillary response, nearly as sensitive as visual evoked responses, but less sensitive than static perimetry. The sensitivity of the Pulfrich stereo-illusion in detecting delayed nerve conduction has been further attested to by the demonstration of this phenomenon in patients with multiple sclerosis lacking both historical and clinical evidence of optic nerve lesions.

My invention resides in the construction of a relatively inexpensive device which utilizes the Pulfrich stereoillusion phenomenon. The device not only controls the variables of the phenomenon but also contains a fixation point which allows easy and reliable perception of the stereo-illusion. This device makes it possible for all normal sighted people to perceive visual phenomenon. Consequently, the inability to perceive the stereo-illusion signals the presence of optic nerve or ocular disease. This enables the trained tester to readily detect diseases affecting central vision. Among the conditions which can be detected using the device of the present invention are amblyopia (lazy eye), demyelinating optic neuropathy, compressive optic nerve diseases, macular diseases, and ocular disorders reducing image brightness such as cataract and unequal pupils. In addition, the device in conjunction with adjustable polarizing filters can measure the abnormal stereo-illusion in those people with diseases causing the spontaneous perception of the stereo-illusion.

The use of the device of the present invention may be more readily appreciated by understanding that a person with normal vision will observe, when my device is in operation, the sphere traversing behind the cone. When this has been established, the second step involves an attempt to induce the physiologic stereo-illusion by diminishing vision in first one eye with about 80% light attenuation and then in the other eye in order to produce a sensation of the sphere rotating first either clockwise or counter clockwise around the cone, and then the opposite way when the second eye is subjected to approximately 80% light attenuation. The light attenuation can be quantified by using polarizing filters.

In order to see the stereo-illusion with this device the person requires 20/60 or better acuity in each eye and stereopsis of at least 3000 seconds of arc.

If when either eye has been subjected to approximately 80% light attenuation the person being tested does not see the Pulfrich stereo-illusion phenomena of arcing of the sphere with respect to the cone, this is indicative of diseases reducing visual acuity below 20/60 or stereopsis below 300 seconds of arc.

According to the present invention, I have constructed a device utilizing the Pulfrich stereo-illusion phenomena which comprises a suitably sized sphere and a suitably sized cone, one of which is movable and the other of which is stationary. The sphere and cone are suitably illuminated and positioned with the sphere behind the cone so that they can be seen at eye level by the person to be tested. The sphere traverses slightly behind the cone or the cone moves in the horizontal plane slightly in front of the sphere at a fixed rate of movement. Neutral density filter or polarizing filters capable of being adjusted with respect to the amount of transmitted light are positioned to enable the person to be tested to look through at a distance and readily see the sphere and cone.

According to one embodiment of the present invention, the cone is fixed and the sphere moves, such as, for example, by use of a pendulum or other type motive means, such as magnetic, which will provide an arcing movement to the sphere. Alternately, the sphere may be fixed while the cone moves in an arc slightly in front of the sphere. When the cone is fixed and the sphere moves, the sphere moves slightly behind the cone. In both cases, the sphere is at a height slightly below the peak of the cone.

In a further embodiment of the present invention, a suitable background is provided which further assists the person to be tested in seeing the sphere and cone. If desired, the sphere may be a circle painted on a suitable plate which may be fixed while the cone is as explained above movable with respect thereto.

The adjustable filters preferably comprise two polarizing filters having adjustable light attenuation means which may be set into a suitable device which can have means for supporting the chin of the person and which is adjustable with respect to height to comfortably fit people of different height who will be seated slightly behind the filter support. When in position, the center of the filter should be at approximately the same level as the peak of the cone.

According to a further embodiment of the present invention, a video recording is made of any of the embodiments of my Pulfrich stereo-illusion portion of the present invention. This video recording displays the relative movement between the cone and sphere under suitable illumination. This video recording is then played before a person to be tested who uses the adjustable filters as above described in the same manner as if one of my devices were actually there before him in operation.

The present invention also includes a method of diagnosing optic nerve and ocular disorders in people by utilizing the device of the present invention. An experienced tester is then able to determine, based on the responses received from the person to be tested, whether or not the person is suffering from a disease affecting central vision. If the presence of such a disease is detected using this device, the person is then referred for appropriate medical evaluation of the disease or dysfunction.

With respect to the drawings, Figs 1-11 describe embodiments of my invention.

Fig. 1 shows one embodiment of my device.

Fig. 2 shows a suitable housing structure for the pendulum and cone of my invention.

Fig. 3 is a section taken along the lines 3/3 of Fig. 2.

Fig. 4 is a side view of the enclosure of Fig. 2 taken along lines 4/4 of Fig. 2.

Fig. 5 is an enlarged view of the sphere and cone of the embodiment shown in Fig. 2.

Fig. 6 is an enlarged view of the polarizing filters of the device of the present invention.

Fig. 7 shows an alternate embodiment of the present invention wherein the sphere is stationary and the cone moves in an arc slightly in front of the sphere

Fig. 8 shows a further embodiment of the present invention wherein the sphere is stationary and is depicted on a suitable background structure and the cone moves in an arc as described with respect to Fig. 7.

Fig. 9 shows a further embodiment of my invention as seen by the patient. This is a portable size.

Fig. 10 is an open side view of the embodiment of Fig. 9 showing the use of white lighting and a stationary sphere with a cone movable in an arc by magnetic motive means.

Fig. 11 shows a further embodiment of the present invention wherein a television and video cassette recorder are substituted for one of my devices and are used in combination with the filters.

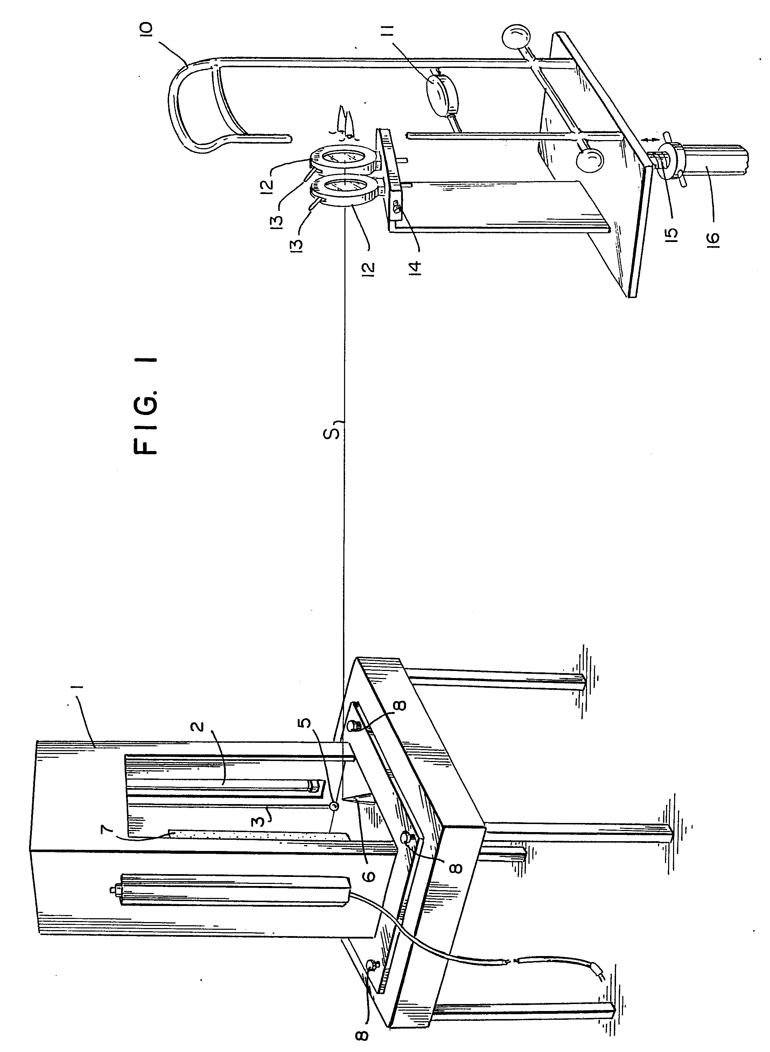

Referring more particularly to the drawings, Fig. 1 shows an enclosure 1 having an illuminating source 2, for example two 40 watt fluorescent blubs on either side, a pendulum 3, a black sphere 5 and a black cone 6, and a white panel 7. Levelling screws 8 are provided to level the housing. This enclosure is set a suitable distance away from the second portion of the device of the present invention which comprises suitable means 10 for enabling the patient to be tested including chin rest 11, polarizing filters 12, adjustments 13 which allow the degree of light to be attenuated to the filters up to 100% and height adjusting means 15 secured to a base 16. Screws 14 are used to adjust the filters 12 to accomodate different interpupillary distances. The center of polarizing filters 12 is positioned so that in use the center will be in the same horizontal plane as the tip of the cone 6. Preferably, the cone is about 5 cm high and the sphere is about 12 mm in diameter. The tip of the cone lies in a vertical plane perpendicular to the line of sight S of the person viewing the sphere and cone, while the outer surface of the sphere closest to the person being treated is tangent to a vertical plane that is also perpendicular to the line of sight S. Line of sight S passes through the center of the polarizing filters.

The sphere is located behind and below the tip of the cone as viewed by the person being tested. Preferably, the distance between the planes is about 5mm and the top of the sphere is preferably about 2mm below the tip of the cone when the cone is moving and the sphere is stationary. However, the precise positioning of the sphere in relation to the tip of the cone is not critical. When the sphere is moving in relation to the cone is stationary, it is preferred that the bottom of the sphere is about 2mm below the tip of the cone as shown.

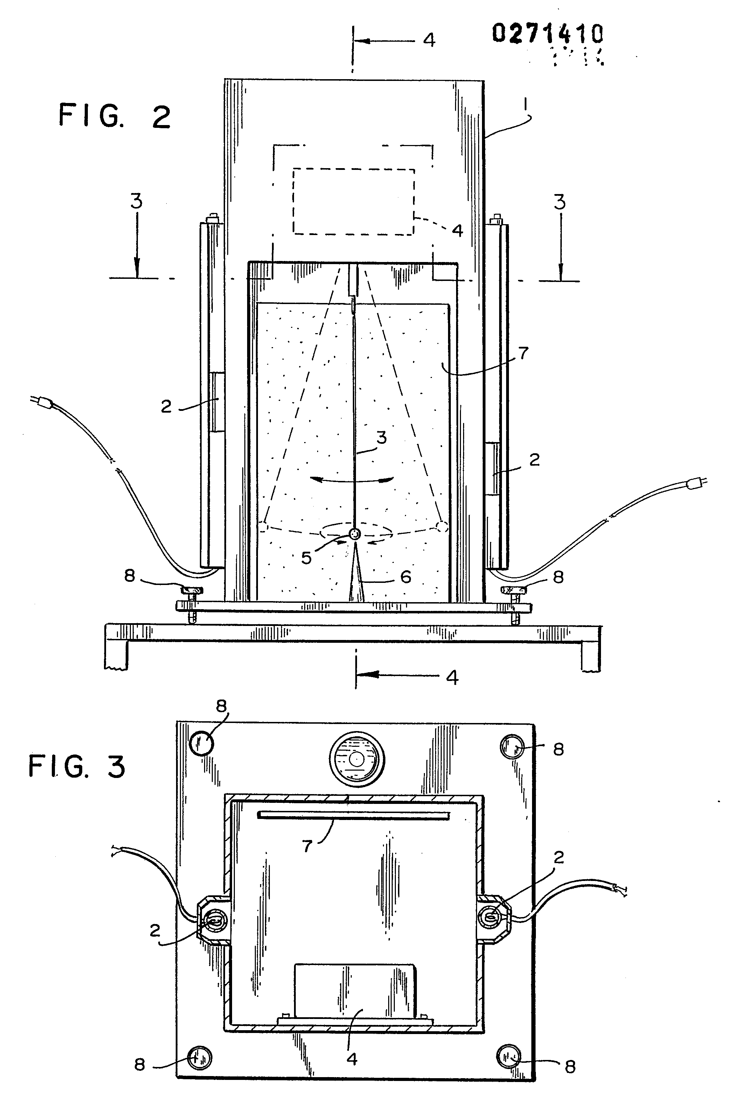

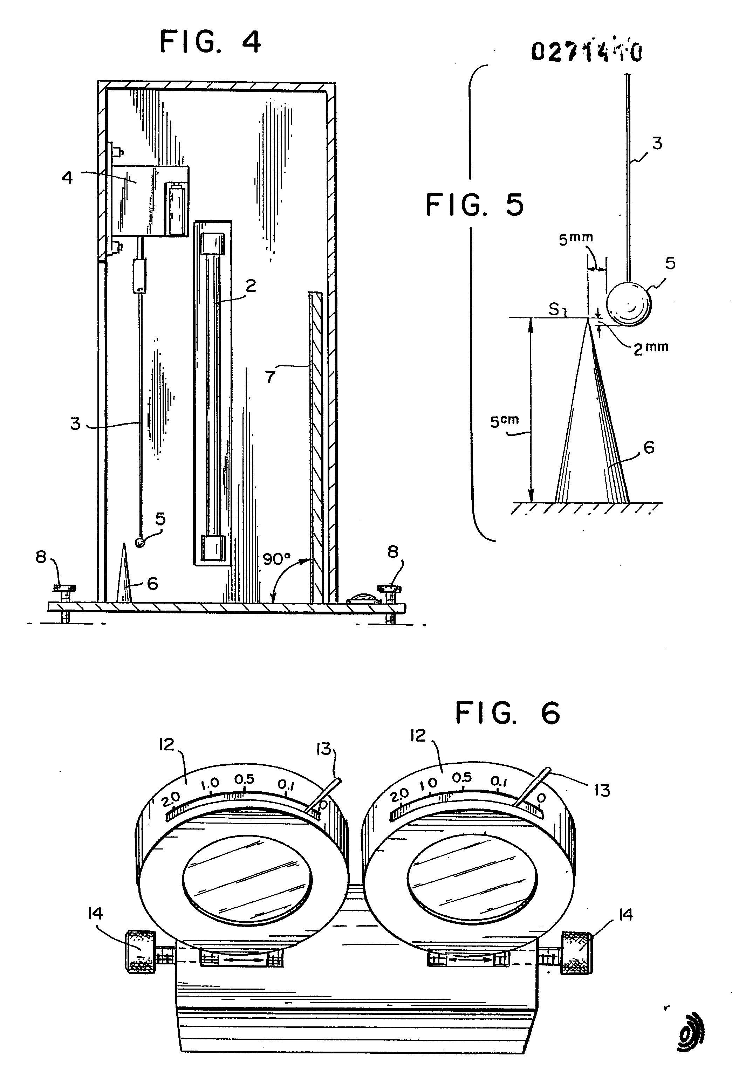

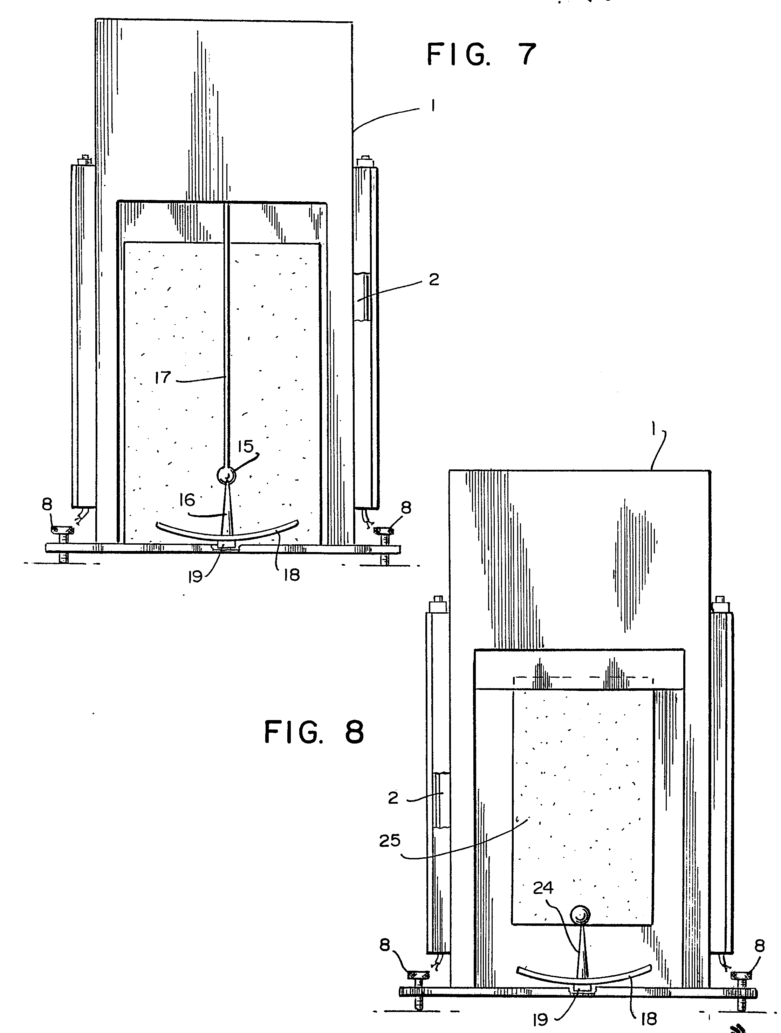

In the embodiment according to the present invention as shown in Figs. 1-6 where there is means for arcing the sphere with respect to the stationary cone, such means may be, for example, a pendulum motor 4 (see Fig. 4). In the embodiment where the sphere is fixed, such as on a rod or other rigid device 17 (see Fig. 7) and the cone 6 moves along arc 18, the cone 16 may be moved by any suitable motive device such as, for example, a magnet or magnetized motive means 19.

In the embodiment of Fig. 8, the sphere 24 is depicted on a suitable background such as a rectangular plate 25 which is affixed in the same position relative to the come as in the embodiment of Figs. 1-6. According to this embodiment, the cone 24 may be moved in an arc as explained above.

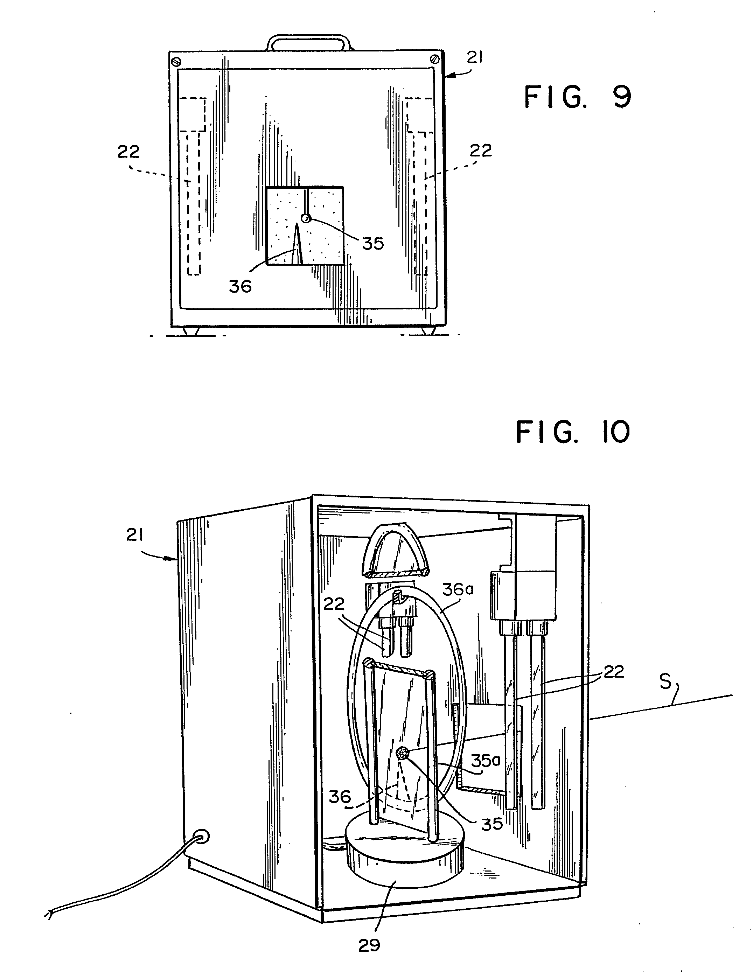

In the embodiment of Figs. 9 and 10 the person views the sphere 35 and cone 36 through an open space at the front of enclosure 21. Suitable lighting such as white light 22 (cold) may be used. The sphere 35 is painted on a transparent material, such as plastic, held in frame 35a, while the cone 36 is mounted on a pendulum 36a, the base 29 of which contains the necessary motive means to oscillate the pendulum 36a. Pendulum 36a is supported by a pivot extending from the transparent material held by frame 35a. Frame 35a, pendulum 36a and base 29 are available commercially as a unit in the form of an electric clock.

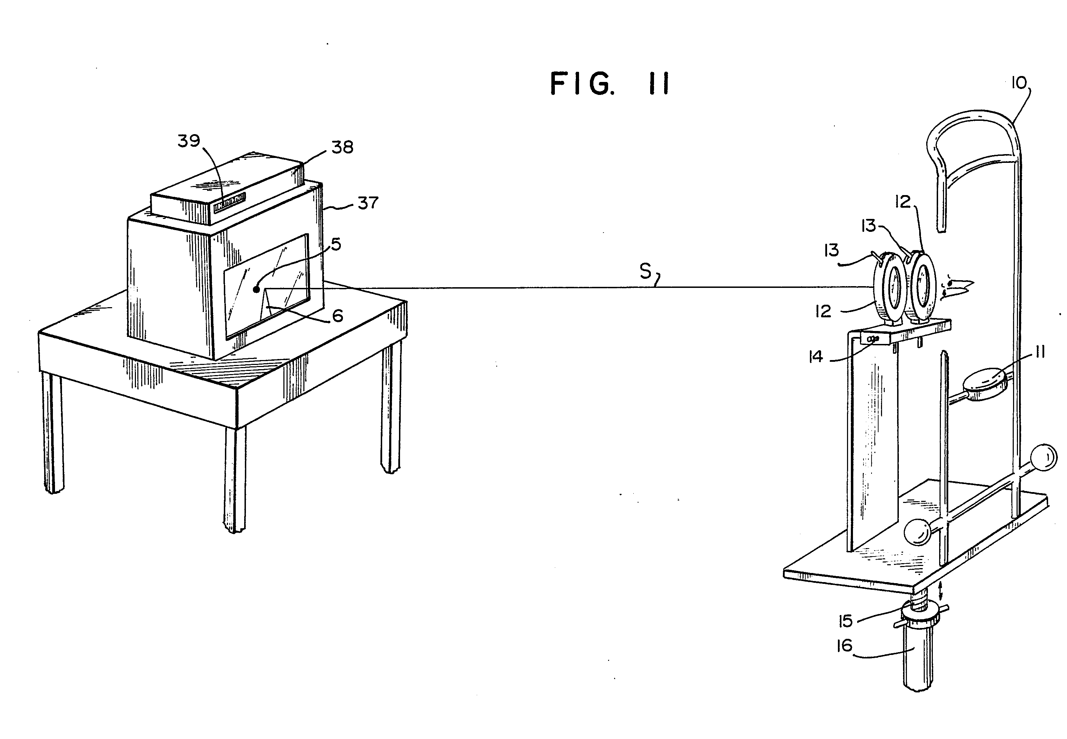

In the embodiment of Fig. 11, one of my devices is replaced by television monitor 37 having a video cassette recorder (VCR) 38 connected thereto. Video cassette 39 may be a video recording of the cone and sphere of one of my Pulfrich stereo-illusion devices in operation as above described, or it may be any other recording of the relative movement between a cone and sphere as described above. This video recording, made under suitable illumination, is played through the television monitor to the person to be tested who uses the filters in the same manner as when one of my Pulfrich stereo-illusion devices is in operation before him. The distance S in Fig. 11 is the same as that of Fig. 1.

While the polarizing filter section of the device is preferably spaced at a suitable viewing distance from the enclosure section of the instant invention, I have found that a particularly optimal distance is between about 1.5 and about 3.0 meters wherein the visual stereoptic effect is maximized.

The device of my invention is a diagnostic tool for detection of diseases which interfere with equal binocular vision. The testing procedure is simple and quick, as explained above. Children four years of age can reliably complete the test in two minutes. Since the test is simple, quick, and reliable, large populations can be readily screened for diseases which interfere with vision or impair optic nerve function.

For example the test could be performed on all first grade students to detect disorders that would otherwise require formal ophthalmologic examination to include acuity, stereopsis, eye muscle coordination, visual evoked potention testing.

In addition to be useful as a screening test, my device is useful:

1) To detect optic nerve involvement in patients suspect of having neurologic disease. The device would be used in the way the visual evoked potential test is used to measure optic nerve impairment;

2) To quantitate visual or optic nerve impairment by adjustable polarizing filters in order to follow the progression or improvement of the disease process; and

3) To test for malingerer who claims vision loss.

Other and further modifications of my device and uses will be more fully appreciated by those skilled in the visual and neurophysiologic sciences.

## Claims

1. A device useful in diagnosing optic nerve and ocular diseases in people who are capable of experiencing the Pulfrich stereo-illusion phenomenon, which comprises a suitably sized sphere (5) and a suitably sized cone (6), one of which is movable and the other of which is stationary, said sphere and said cone being suitably illuminated and positioned with the bottom of the sphere below the cone so that they can be seen by a person to be tested, means either for traversing the sphere slightly behind the cone or moving the cone in an arc slightly in front of the sphere at a fixed rate of movement, polarizing filters (12) capable of being adjusted with respect to the amount of light which will pass through disposed in adjustable means (15) suitable for the person to be tested to look through at a distance from said sphere and cone enabling said person to readily see said sphere and cone.

2. A device according to claim 1 having means for moving said sphere in an arc slightly behind said cone and wherein said cone is stationary.

3. A device according to claim 1 having means (19) for moving said cone (16) in an arc slightly in front of said sphere, said sphere (15) being stationary.

4. A device according to claim 1 wherein said sphere (5) and said cone (6) are disposed within a structure (1) having a suitable background (7) to facilitate the person to be tested seeing the cone and sphere under operative conditions.

5. A device according to claim 1 having means for moving the said cone in an arc slightly in front of said sphere and wherein said sphere is a solid color circle depicted on a suitable background.

6. A device according to claim 1 having two polarizing filters (12) disposed within means having a person chin supporting means (11) and height adjustable means (15, 16).

7. A device according to claim 1 having means for movably positioning said polarizing filters at a level with respect to the person to be tested so that the appriximate center of the filter when placed in proximity to the eyes of the person to be tested is in the same horizontal plane as the tip of the cone.

8. A device useful in diagnosing optic nerve and ocular diseases in people who are capable of experiencing the Pulfrich stereo-illusion phenomenon, which comprises a video recording (38) of the relative movement between a cone (6) and sphere (5) according to the Pulfrich stereo-illusion phenomenon, means (37) for playing said recording in front of a person to be tested, polarizing filters (12) capable of being adjusted with respect the amount of light which will pass through disposed in adjustable means suitable for the person to be tested to look through at a distance from said sphere and cone enabling said person to readily see said sphere and cone.

9. A method of diagnosing optic nerve and ocular diseases in people who are capable of experiencing the Pulfrich stereo illusion phenomenon which comprises placing a person to be tested behind the filters of the device of any one of claims 1-8, illuminating said sphere and said cone, activating said means, moving one of said sphere and said cone, determining if said person sees the sphere traversing behind the cone with both eyes with full vision, diminishing the vision in the first eye by adjusting the filter to achieve about 80% light attenuation, determining how the person sees the sphere in relation to the cone, restoring full vision to the first eye, diminishing the vision in the other eye by adjusting the filter to achieve about 80% light attenuation and determining how the person sees the sphere in relation to the cone through the second eye.

FIG. I

FIG. 2

0271410

FIG. 3

## FIG. 4

## FIG. 5

0271410

## FIG. 6

**FIG. 7**

1

2

17

15

16

8

19

18

8

**FIG. 8**

1

2

25

24

8

8

19

18

0271410

FIG. 9

FIG. 10

# FIG. 11